# EUROPEAN PATENT SPECIFICATION

(11) **EP 2 039 387 B1**
(45) Date of publication and mention of the grant of the patent: **11.01.2012**
(21) Application number: 07425585.2
(22) Date of filing: 24.09.2007
(51) Int. Cl.: A61M 16/10, A61M 16/16

(54) **System for conditioning respiratory gases**
System zur Aufbereitung von Atemgas
Système pour le conditionnement de gaz respiratoires

(43) Date of publication of application: 25.03.2009
(73) Proprietor: Covidien AG, 8212 Neuhausen am Rheinfall (CH)
(72) Inventor: Zucchi, Giuseppe, 41039 San Possidonio (IT); Borali, Stefano, 41037 Mirandola (IT); Tralli, Stefano, 46022 Felonica (IT)
(74) Representative: Jorio, Paolo

(56) References cited:
- WO-A-2006/127257
- WO-A-2007/068132
- DE-A1- 2 950 747
- US-A- 5 482 031

## Description

### FIELD

The present invention relates to a system of conditioning respiratory gases.

The system is intended for use in Intensive Care to provide the right moisture/temperature level of gases inhaled by intubated patients under artificial ventilation.

The present invention may be used to particular advantage, though not exclusively, in Anaesthesiology and Intensive Care Unit (ICU), to which the following description refers purely by way of example.

### PRIOR ART

At present, the respiratory tracts of intubated patients under artificial ventilation in Intensive Care Units are heated and humidified using two main methods, depending on how long the patient is expected to be kept in Intensive Care.

A first passive conditioning system employing a heat and moisture exchanger (HME) is used when the patient is expected to remain in Intensive Care for roughly less than 72 hours.

As is known, an HME operates by retaining moisture and heat from the gases exhaled by the patient, and yielding most of the retained moisture and heat to the patient at the next inhalation stage.

Devices of this sort are certified to supply patients with an absolute moisture level of 28 to 33 mg/l, at a temperature ranging between 28 and 31°C, and to maintain correct respiration physiology for roughly 72 hours' treatment.

Operation of these devices normally remains stable for 24 hours, after which, the patient may experience difficulty in breathing (increase in work of breathing, WOB) caused by an increase in flow resistance, thus justifying replacement of the device every 24 hours.

A second respiratory gas conditioning system is based on active humidification.

The best currently marketed device provides for heating and humidifying gas supply to the patient to an absolute moisture level of 40 mg/l or more, and a temperature ranging between 35 and 39°C, and calls for very little maintenance, by temperature-regulating the expiration conduit to eliminate condensation.

An intermediate active device, operating in combination with an HME, however, provides for increasing heat and moisture supply to the patient by compensating the inhaled gas with a few mg of water vapour, thus enabling longer-term operation of the device (over 72 hours).

The HME system has the following advantages:
- less maintenance than an active device;
- adequate maintenance of correct respiration physiology for 72 hours;
- is easy to use;
The HME system may cause:
- "poor" humidification in most cases;
- augmentation of dead space within the respiratory circuit;
- eventual aumentation in flow resistance, due to potential clogging (build-up of condensation) of the heat-exchange element.

The active-humidifier system has the following advantages:
- higher moisture supply as compared with a passive device;
- longer-term operation as compared with a passive device.
   The active-humidifier system may cause:

- possible over-humidification, caused by incorrect setting of the humidifier;
- high cost of the disposable-cartridge or -can circuit and sterile water;
- more frequent monitoring required than with passive devices;
- the moisture-sensitive flow sensors of the ventilator may have to be changed more frequently than usual, due to build-up of condensation on the expiration side, thus increasing operating cost;
- high consumption of sterile water.

A moisture-enrich HME device has the following advantages:
- higher moisture supply as compared with a passive device;
- consumes less sterile water than an active device;
   A moisture-enrich HME device may cause:
- additional bulk and weight of the HME, which are undesirable close to the patient.
   Various systems of the above types are described in WO2006/127257 (DHUPER et al.), which discloses the features of the preamble of claim 1.

One embodiment in the above document, described with reference to Figures 4-6, employs an HME remote from the patient and combined with a number of temperature-regulated conduits.

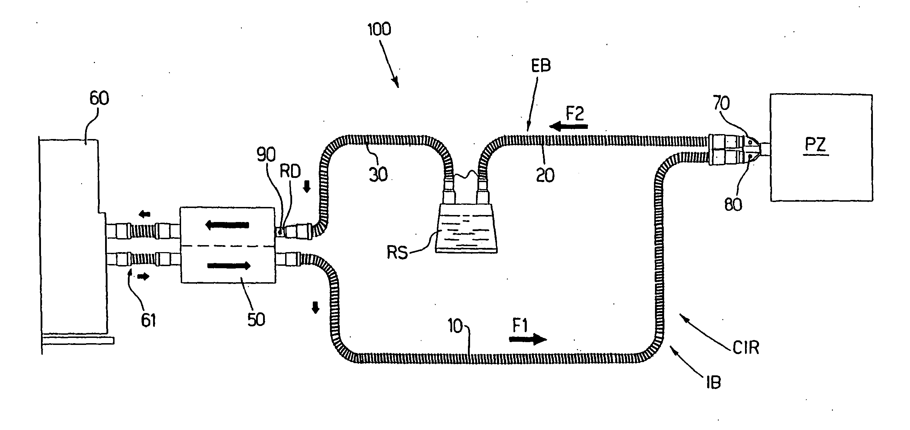

Another embodiment, shown in Figures 1-3, employs a device for injecting drugs into the device. Alternatively, an atomizer may be used.

Though successful, the systems described in WO2006/127257 have proved unreliable as regards precise regulation of the moisture level of the gas inhaled by the patient.

### SUMMARY

It is therefore an object of the present invention to ensure correct moisture supply to the patient. As is known, in this field, the basic parameters of the gas are moisture (i.e. the amount of water vapour per unit volume of gas) and temperature.

The main characteristic of the respiratory gas conditioning system according to the invention lies in combining operation of a passive HME (located close to the ventilator, and characterized by inducing very little dead space in the system) with that of an active heating and moisture-enriching device comprising one or more water reservoirs (possibly heated) and preferably two temperature-regulated conduits.

### DETAILED DESCRIPTION

A non-limiting embodiment of the present invention will be described by way of example with reference to the attached drawing.

As shown in the attached drawing, the system 100 according to the present invention comprises:
- three temperature-regulated conduits 10, 20, 30, one for an inhale branch IB, and two for an exhale branch EB;
- a water reservoir RS containing water possibly heated by an electric resistor (not shown), and which has top-up access, is characterized by containing a small amount of water, and is located along exhale branch EB;
- a heat and moisture exchanger (HME) 50, which is characterized by strict separation of the inhale flow F1 and exhale flow F2, is located close to a ventilator 60, and provides for separating the inhale/exhale flows while still ensuring correct heat and moisture exchange between the two, and with no increase in dead space in the circuit;
- a Y piece connector 70, which is located close to a patient PZ, connects the patient PZ to inhale branch IB and exhale branch EB, and has a socket for a temperature sensor 80 on inhale branch IB;
- a straight connector RD, with a socket for a temperature sensor 90, to connect HME 50 to exhale branch EB; and
- a thermostat (not shown) for the three temperature-regulated conduits 10, 20, 30. The term "thermostat" is intended herein to mean an electronic central control unit (not shown) connected electrically to temperature-regulated conduits 10, 20, 30 and temperature sensors 80, 90 to regulate the temperature of the gas flow to/from the patient PZ.

System 100 operates as follows:

Gas is exhaled by the patient PZ at roughly 32°C, and, as it flows along temperature-regulated exhale branch EB, is heated to a higher temperature, so as to be further enriched with moisture as it flows over the surface of the water inside reservoir RS.

The gas is then heated further, and is heated and humidified by the time it reaches HME 50 (close to ventilator 60) where the heat and moisture gradient assists release of heat and moisture to HME 50 itself.

Assuming a high-performance HME 50 is used, enough heat and moisture is retained by the exchanger to supply ventilator 60 with relatively dry gas, and so eliminate the condensation well on the exhale line.

This therefore eliminates any problems with the moisture-sensitive flow sensor (not shown) forming part of ventilator 60.

At the next inhalation stage, the dry gas flowing through HME 50 from ventilator 60 is charged with heat and moisture and fed to the patient PZ along temperature-regulated inhale branch IB, which maintains the temperature of the gas to prevent the moisture in the gas from condensing.

In other words, the amount of heat and moisture in the gas supply to the patient PZ is controlled by adjusting the temperature of the gas flowing along inhale branch IB and exhale branch EB.

By determining the temperature of the gas supply to the patient PZ by means of temperature sensors 80, 90 installed along the circuit, the temperature of temperature-regulated conduits 10, 20, 30 can be controlled by a thermostat (not shown) as required by the patient PZ.

More specifically, heating temperature-regulated conduit 20 heats the gas exhaled by the patient PZ to gather more moisture from water reservoir RS; while heating the gas in temperature-regulated conduit 30 maintains the temperature and moisture level of the gas, and also produces a sufficient gradient between exhale branch EB and HME 50 to ensure effective transfer of heat and moisture to the exchange element (not shown) of HME 50. The exchange element, in turn, having a much higher heat and moisture content than the incoming gas from ventilator 60, heat and moisture are transferred to the inhale flow (F1) to the patient PZ, the conditions of such inhale flow (F1) are maintained along inhale branch IB by temperature-regulated conduit 10.

The main advantages of the respiratory gas conditioning system according to the present invention are as follows:

- low energy consumption as compared with a conventional active humidifier; energy, in fact, is only used to heat the temperature-regulated conduits and possibly slightly heat the water reservoir;

- low water consumption as compared with a conventional active humidifier; the system, in fact, only supplies the amount of moisture needed to compensate moisture loss by the patient exhaling;

- very few routine checks, thus reducing system maintenance as compared with both passive and active devices;

- elimination of conventional system water traps; by virtue of the high performance of the HME, the exhale-side gas is dry enough to eliminate the condensation well; and calibrating moisture content to simply compensate consumption prevents the formation of surplus moisture, and so eliminates the need for a condensation well along the inhale branch;

- longer-term operation of the system as compared with a conventional HME;

- adequate heating and humidification of the patient-inhaled gases; the amount of moisture added by the enrich system, in fact, compensates the moisture loss of the HME, thus supplying the patient with the required moisture level;

- improved patient safety; the low power employed and the small amount of added moisture safeguard the patient against scalding and surplus moisture;

- complete separation of the inhale and exhale flows by the HME enables elimination of one-way valves from the circuit;

- lighter weight of the circuit close to the patient; unlike other humidifiers, the HME is located close to the ventilator, as opposed to the patient; and eliminating the water traps, which fill up with water, further reduces the weight of the circuit weighing on the patient;

- the system is also ideal for use with new-born babies, being so flexible and effective to humidify and heat even small gas flows by simply increasing temperature regulation of the conduits.

## Claims

1. A respiratory gas conditioning system (100) comprising:
- a heat and moisture exchanger (HME) (50); and
- an inhale branch (IB) and an exhale branch (EB); each of said inhale branch (IB) and exhale branch (EB) being connected to said HME (50) and, by means of a connector (70), to a patient (PZ); said HME (50) being located close to a ventilator (60)
the system (100) being **characterized in that** it comprises a water reservoir (RS) connected to said exhale branch (EB) and located upstream from said HME.

2. A system (100) as claimed in Claim 1, wherein said water reservoir (RS) is connected upstream to a first temperature-regulated conduit (20) and downstream to a second temperature-regulated conduit (30).

3. A system (100) as claimed in Claim 2, wherein said water reservoir (RS) contains a given amount of water, the surface of which is swept by the exhaled gas flowing from the first temperature-regulated conduit (20) to the second temperature-regulated conduit (30).

4. A system (100) as claimed in any one of the foregoing Claims, wherein the water in the water reservoir (RS) is heated by an electric resistor.

5. A system (100) as claimed in Claim 1, wherein
it comprises an electronic device for temperature-regulating the whole system.

## Patentansprüche

1. Atemgasaufbereitungssystem (100), enthaltend:
- einen Wärme- und Feuchtigkeitstauscher (HME) (50); und
- einen Inhalierzweig (IB) und einen Exhalierzweig (EB), wobei jeder Inhalierzweig (IB) und Exhalierzweig (EB) mit dem HME (50) verbunden ist und mittels eines Verbinders (70) mit einem Patienten (PZ) verbunden ist;
wobei der HME (50) nahe an einem Ventilator (60) angeordnet ist; wobei das System (100) **dadurch gekennzeichnet ist, dass** es ein Wasserreservoir (RS) aufweist, welches mit dem Exhalierzweig (EB) verbunden ist und dem HME vorgeschaltet angeordnet ist.

2. System (100) nach Anspruch 1, wobei das Wasserreservoir (RS) mit einer ersten temperaturregulierten Leitung (20) vorgeschaltet und mit einer zweiten temperaturregulierten Leitung (30) nachgeschaltet verbunden ist.

3. System (100) nach Anspruch 2, wobei das Wasserreservoir (RS) eine vorgegebene Wassermenge enthält, dessen Oberfläche von dem exhalierten Gas überstrichen wird, welches von der ersten temperaturregulierten Leitung (20) zu der zweiten temperaturregulierten Leitung (30) strömt.

4. System (100) nach einem der vorangehenden Ansprüche, wobei das Wasser in dem Wasserreservoir (RS) von einem elektrischen Widerstand erhitzt wird.

5. System (100) nach Anspruch 1, wobei es eine elektronische Vorrichtung zur Temperaturregulierung des gesamten Systems aufweist.

## Revendications

1. Système (100) de conditionnement de gaz respiratoires comprenant :
- un échangeur de chaleur et d'humidité (ECH) (50), et
- une branche d'inhalation (IB) et une branche d'exhalation (EB) ; chacune desdites branche d'inhalation (IB) et branche d'exhalation (EB) étant connectée audit ECH (50) et à un patient (PZ) au moyen d'un connecteur (70), ledit ECH (50) étant situé près d'un ventilateur (60) ;
le système (100) étant **caractérisé en ce qu'**il comprend un réservoir d'eau (RS) connecté à ladite branche d'exhalation (EB) et situé en amont dudit ECH.

2. Système (100) selon la revendication 1 dans lequel ledit réservoir d'eau (RS) est connecté en amont à un premier conduit thermo-régulé (20) et en aval à un deuxième conduit thermo-régulé (30).

3. Système (100) selon la revendication 2 dans lequel ledit réservoir d'eau (RS) contient une quantité donnée d'eau, dont la surface est balayée par le gaz exhalé s'écoulant du premier conduit thermo-régulé (20) vers le deuxième conduit thermo-régulé (30).

4. Système (100) selon l'une quelconque des revendications précédentes dans lequel l'eau du réservoir d'eau (RS) est chauffée par une résistance électrique.

5. Système (100) selon la revendication 1, qui comprend un dispositif électronique pour thermo-réguler le système entier.
